# EUROPEAN PATENT SPECIFICATION

(11) **EP 4 205 571 B1**
(45) Date of publication and mention of the grant of the patent: **09.12.2026**
(21) Application number: 22214072.5
(22) Date of filing: 16.12.2022
(51) Int. Cl.: A24F 40/05, A61M 11/00, A61M 15/00, B05B 17/06

(54) **ULTRASONIC ATOMIZATION ASSEMBLY AND ULTRASONIC ATOMIZATION DEVICE**
ULTRASCHALLZERSTÄUBUNGSANORDNUNG UND ULTRASCHALLZERSTÄUBUNGSVORRICHTUNG
ENSEMBLE D'ATOMISATION ULTRASONORE ET DISPOSITIF D'ATOMISATION ULTRASONORE

(30) Priority: 29.12.2021 CN 202111642024
(43) Date of publication of application: 05.07.2023
(73) Proprietor: Shenzhen Moore Vaporization Health & Medical Technology Co., Ltd., Shenzhen, Guangdong (CN)
(72) Inventor: TANG, Ming, Shenzhen (CN); ZHU, Yongxiong, Shenzhen (CN); OUYANG, Guanglin, Shenzhen (CN)
(74) Representative: Herrero & Asociados, S.L.

(56) References cited:
- US-A1- 2013 112 770
- US-A1- 2021 052 014
- US-B2- 9 452 441

## Description

### TECHNICAL FIELD

The present disclosure relates to the field of the ultrasonic atomization technology, and in particular to an ultrasonic atomization assembly and an ultrasonic atomization device.

### BACKGROUND

With the development of medical technology, an aerosol inhalation administration method has been accepted by more and more people due to advantages of low damage and high efficacy. Ultrasonic microporous atomization administration is a rapidly developing administration method.

An existing ultrasonic microporous atomization assembly is mainly composed of a piezoelectric ceramic ring and a microporous atomizer plate. A main working principle is to use inverse piezoelectric effect of the piezoelectric ceramic ring to generate high frequency micro-oscillation, so as to split a surface of an aerosol-generating substrate. Thus, aerosol of tiny particles with vector property is formed, and then disperses in the form of mist.

However, the existing ultrasonic microporous atomization assembly has large invalid vibration loss, high working noise and serious heating problems; Moreover, the small-particle-size aerosol accounts for a low proportion in the atomized aerosol.

US patent application Pub. No. US2021052014A1 provides an aerosol delivery device that may include a housing defining an outer wall and further including a power source and a control component. The device also includes a mouthpiece portion that defines an exit aerosol path, a tank portion that includes a reservoir configured to contain a liquid composition, and an atomization assembly configured to vaporize the liquid composition to generate an aerosol. The atomization assembly includes a mesh plate and a vibrating component, wherein the mesh plate and the vibrating component are configured to be separable from each other at a detachable interface. The detachable interface may be located at various locations of the device, including between the mouthpiece portion and the tank portion, within the mouthpiece portion, within the tank portion, within a separable atomization assembly, within a cartridge, within a control unit, or between a cartridge and a control unit.

US patent application Pub. No. US2013112770A1 provides a nebulization structure. The nebulization structure includes a driving element, a structure plate and a nebulization plate. The structure plate is installed on a side of the driving element, and the structure plate is substantially in a circular disc shape and has a plurality of through holes, and at least one rib is formed between adjacent through holes to define a water guide passage. The nebulization plate is clamped between the driving element and the structure plate and made of a polymer material to overcome the problems of having easy metal fatigue and embrittlement of the nebulization plate made of metal, and being corroded by corrosive liquids.

### SUMMARY

An ultrasonic atomization assembly and an ultrasonic atomization device provided by the present disclosure, aimed to solve problems that the existing ultrasonic atomization assembly has great invalid vibration loss, high working noise, serious heat generation, and relatively low proportion of small-particle-size aerosol in atomized aerosol.

In order to solve the above technical problems, a first technical solution provided in the present disclosure is providing an ultrasonic atomization assembly, a second technical solution in the present disclosure is providing an ultrasonic atomization device, and the ultrasonic atomization assembly and the ultrasonic atomization device according to the present invention are as set out claims 1-10.

The embodiments of the present disclosure has the following beneficial effect: Different from the prior art, the embodiments of the present disclosure provide an ultrasonic atomization assembly and an ultrasonic atomization device; in the ultrasonic atomization assembly, a loading board is added and placed on a side surface of an atomizer plate away from an piezoelectric drive element, an overall structural rigidity of the atomizer plate is effectively improved, so that a vibration mode of the atomizer plate is effectively restricted, thus not only reducing invalid vibration of the atomizer plate and vibration loss caused by the invalid vibration, but also greatly reducing noise caused by the invalid vibration of the atomizer plate and a temperature rise of the atomizer plate. Meanwhile, the proportion of small-size particles in atomized particles is effectively increased. Besides, a ratio of the aperture of a second through hole to a diameter of a microporous zone is greater than or equal to 0.75 and less than or equal to 1.5, so that the ultrasonic atomization assembly achieves a better noise reduction effect and has less temperature rise. In addition, the added loading board can reduce a clamping force of a housing and the piezoelectric drive element on the atomizer plate after installation of the ultrasonic atomization assembly and the housing, thus effectively reducing vibration performance attenuation caused by the clamping of the atomizer plate. Moreover, the second through hole is provided at a position of the loading board corresponding to the microporous zone, so as to effectively ensure that a liquid inlet surface or a mist outlet surface of the atomizer plate is in communication with the external, and ensure that an aerosol substrate or atomized aerosol particles can pass through smoothly.

### BRIEF DESCRIPTION OF THE DRAWINGS

In order to describe technical solutions in embodiments of the present disclosure more clearly, the drawings for the description of the embodiment will be described in brief. Obviously, the drawings in the following description are only some of the embodiments of the present disclosure. For a person of ordinary skill in the art, other drawings may be obtained based on the following drawings without any creative work.
FIG. 1 is a schematic structural view of an ultrasonic atomization device according to an embodiment of the present disclosure;
FIG. 2 is a structural exploded view of an ultrasonic atomization assembly according to an embodiment of the present disclosure;
FIG. 3 is a vertical sectional view of an atomizer plate according to an embodiment of the present disclosure;
FIG. 4 is a side sectional view of a first embodiment of an ultrasonic atomization assembly with a boss of the present disclosure;
FIG. 5 is a side sectional view of a second embodiment of an ultrasonic atomization assembly with a boss of the present disclosure;
FIG. 6 is a side sectional view of a first embodiment of an ultrasonic atomization assembly without a boss of the present disclosure;
FIG. 7 is a side sectional view of a second embodiment of an ultrasonic atomization assembly without a boss of the present disclosure; and
FIG. 8 is a schematic structural view of a loading board of the present disclosure.

### DETAILED DESCRIPTION

The technical solutions in the embodiments of the present disclosure will be clearly and completely described below in combination with the accompanying drawings of the embodiments of the present disclosure. Obviously, the described embodiments are only part of the embodiments of the present disclosure but not all embodiments.

The terms "first", "second" and "third" in the present disclosure are only configured to description and cannot be understood as indicating or implying relative importance or implying the number of an indicated technical feature. Therefore, the feature defined with "first", "second", and "third" may expressly or implicitly include at least one of the features. In the description of the present disclosure, "many" means at least two, such as two, three and the like, unless otherwise explicitly and specifically defined. All directional indications (such as up, down, left, right, front, back...) in the embodiments of the present disclosure are only configured to explain relative position relations, movement situation and the like between components under a certain posture (as shown in the accompanying drawings). If the certain posture changes, the directional indication also changes accordingly. Besides, the terms "include" and "have" and any variations thereof are intended to cover non-exclusive inclusion. For example, a process, method, system, product or device including a series of steps or units is not limited to the listed steps or units, but optionally also includes unlisted steps or units, or optionally also includes other steps or units inherent to the process, method, product or device.

The "embodiment" mentioned herein means that a specific feature, structure, or characteristic described in combination with the embodiments may be included in at least one embodiment of the present disclosure. The appearances of the phrase in various positions of the specification do not necessarily refer to the same embodiment, nor a separate or an alternative embodiment that is mutually exclusive with other embodiments. Persons skilled in the art can explicitly and implicitly understand that the embodiments described herein may be combined with other embodiments.

The present disclosure is described in detail below in combination with the accompanying drawings and the embodiments.

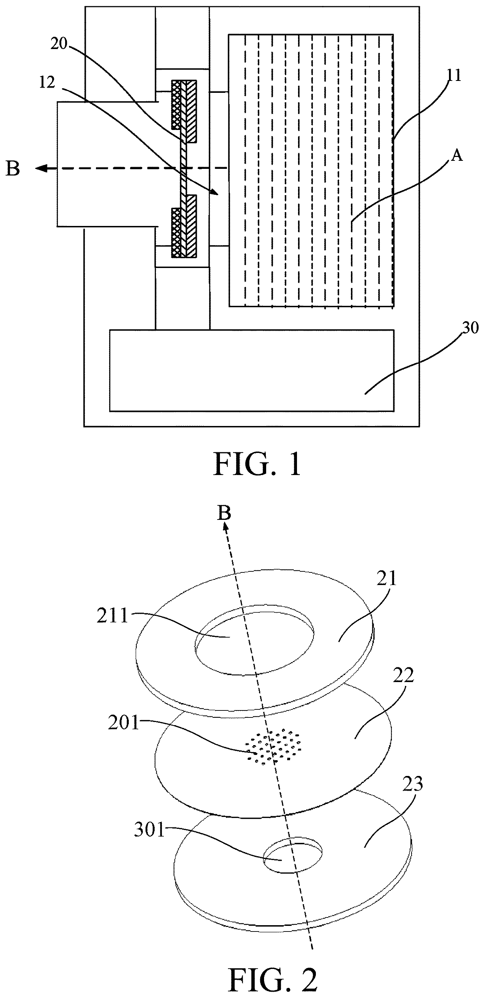

Referring to FIG. 1, FIG. 1 is a schematic structural view of an ultrasonic atomization device according to an embodiment of the present disclosure. In the embodiment, an ultrasonic atomization device is provided, the ultrasonic atomization device can be configured in the technical field of medical treatment, beauty, electronic cigarette and the like. The structure of the ultrasonic atomization device includes a housing 10, an ultrasonic atomization assembly 20, and a power supply assembly 30.

The housing 10 includes a liquid storage cavity 11 and a liquid outlet 12 connected with the liquid storage cavity 11. The liquid storage cavity 11 is configured to contain an aerosol-generating substrate A. The aerosol-generating substrate A is a liquid medicine formed by a certain medicine dispersed in a liquid solvent, e-liquid or any other liquid suitable for the ultrasonic atomization. The ultrasonic atomization assembly 20 is disposed at the liquid outlet 12 of the housing 10, and is configured to generate high-frequency oscillation when power is turned on, so as to atomize the aerosol-generating substrate A. The power supply assembly 30 is electrically connected to the ultrasonic atomization assembly 20, and is configured to supply power to the ultrasonic atomization assembly 20. The power supply assembly 30 may specifically be a lithium-ion battery.

The existing ultrasonic atomization assembly is generally composed of a piezoelectric ceramic ring and an atomizer plate. However, when the existing ultrasonic atomization assembly is working, vibration mode constraint is insufficient and the vibration is not concentrated, which leads to high working noise, serious heating and great invalid vibration loss. In addition, the existing atomizer plate generally has a boss structure. After stamping the boss, the atomizer plate with the boss structure tends to enlarge micro-pores on the atomizer plate, resulting in a larger atomizing particle size of the atomizer plate, which reduces the proportion of small-particle-size aerosol in the atomized aerosol and is not beneficial to atomization inhalation.

The embodiments of the present disclosure provide an ultrasonic atomization assembly 20, where the ultrasonic atomization assembly 20 can effectively constrain the ultrasonic vibration mode of the atomizer plate 22, so that the atomizer plate 22 can vibrate in a concentrated manner, which effectively reduces the invalid vibration of the atomizer plate 22, the working noise of the ultrasonic atomization assembly 20, and the temperature rise. In this way, the atomization amount per unit time of the ultrasonic atomization assembly 20 is increased. Furthermore, the ultrasonic atomization assembly 20 can greatly improve the proportion of the small-particle-size aerosol in the aerosol particles formed by the atomization.

Referring to FIG. 2, FIG. 2 is a structural exploded view of an ultrasonic atomization assembly according to an embodiment of the present disclosure. The ultrasonic atomization assembly 20 includes a piezoelectric drive element 21, an atomizer plate 22, and a loading board 23 that are stacked in sequence.

The piezoelectric drive element 21 is configured to convert electrical energy into mechanical energy by using an inverse piezoelectric effect when power is turned on, so as to generate high frequency vibration. The piezoelectric drive element 21 is provided with a first through hole 211, and the aerosol-generating substrate A can enter the surface of the atomizer plate 22 through the first through hole 211; or the aerosol particles, formed after the aerosol-generating substrate A is atomized by the atomizer plate 22, are scattered through the first through hole 211. In an alternative embodiment, the piezoelectric drive element 21 may be the piezoelectric ceramic ring. A cross section of the piezoelectric drive element 21 may be in the shape of a circle, a ring, a square and the like, which may be selected according to an actual situation.

The atomizer plate 22 comprises a liquid inlet surface 221 and a mist outlet surface 222 that are opposite to each other. The piezoelectric drive element 21 is stacked on the liquid inlet surface 221 or the mist outlet surface 222 of the atomizer plate 22. The atomizer plate 22 has a microporous zone 201, where the microporous zone 201 is configured to break up the aerosol-generating substrate A into small particles under the driving of the piezoelectric drive element 21, and the small particles escape through the microporous zone 201, so as to form the aerosol. The aerosol-generating substrate enters the microporous zone 201 from the liquid inlet surface 221 of the atomizer plate 22, and the scattered small particles escape from the microporous zone 201 from the mist outlet surface 222 of the atomizer plate 22. In the embodiment, the atomizer plate 22 may be a screen mesh; the atomizer plate 22 may be made of materials such as stainless steel, titanium alloy, palladium alloy and the like, which is not limited in the present disclosure. The atomizer plate 22 may be an atomizer plate 22a/22b in the following embodiments.

In the embodiment, the microporous zone 201 of the atomizer plate 22 is disposed corresponding to the first through hole 211, and an aperture of the first through hole 211 may be greater than or equal to a diameter of the microporous zone 201, so as to ensure that all atomized particles of the aerosol-generating substrate A can pass through. Certainly, the aperture of the first through hole 211 may also be slightly smaller than the diameter of the microporous zone 201, which is not limited in the present disclosure. The atomizer plate 22 and the piezoelectric drive element 21 may be connected together by gluing, spot welding, or the like.

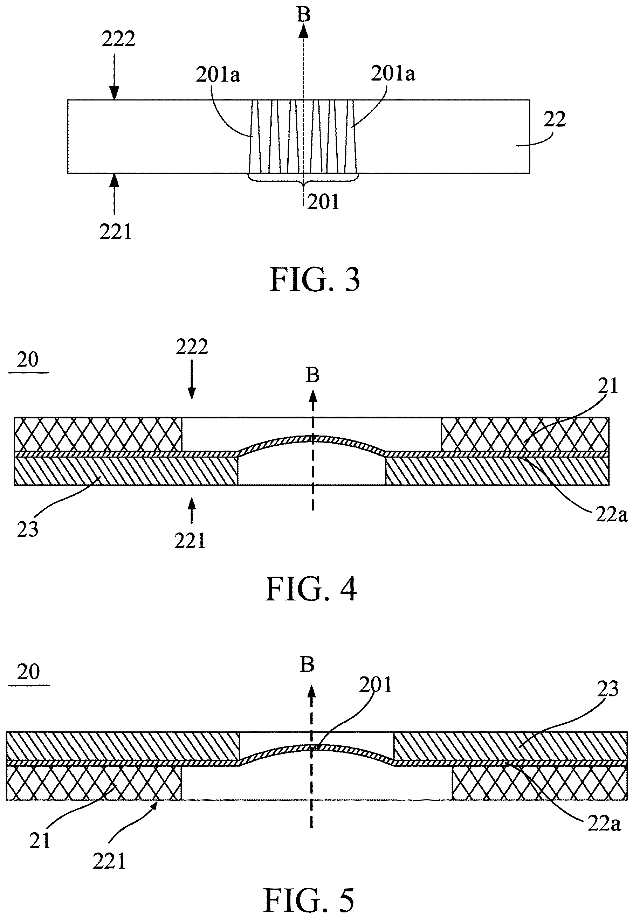

In the embodiment, referring to FIG. 2 and FIG. 3, FIG. 3 is a vertical sectional view of an atomizer plate according to an embodiment of the present disclosure. The microporous zone 201 of the atomizer plate 22 includes a plurality of atomization through holes 201a. The aperture of each of the plurality of atomization through hole 201a gradually decreases along a mist outlet direction B of the ultrasonic atomization assembly 20. In this way, the atomization through hole 201a can be configured to reduce the particle size of the atomized aerosol, so as to improve the proportion of the small-particle-size aerosol in the atomized aerosol, and avoid the problem that an increase in the aperture of the atomization through hole 201a due to the stamping causes a decrease in the proportion of the small-particle-size aerosol in the atomized aerosol can be avoided.

In an alternative embodiment, referring to FIG. 4 or FIG. 5, FIG. 4 is a side sectional view of a first embodiment of an ultrasonic atomization assembly with a boss of the present disclosure; FIG. 5 is a side sectional view of a second embodiment of an ultrasonic atomization assembly with a boss of the present disclosure.

The microporous zone 201 of the atomizer plate 22a raises towards the mist outlet direction B of the ultrasonic atomization assembly 20 to form the boss. In the embodiment, referring to Table 1, compared with the ultrasonic atomization assembly 20a (sample 2) corresponding to the microporous zone 201 of the atomizer plate 22a without the boss, in the case of the same diameter of the microporous zone 201, the atomizer plate 22a with the boss structure provided by the embodiment can increase a surface area of the microporous zone 201 to a certain extent, which means increasing a contact area with the aerosol-generating substrate A, thereby effectively improving the atomization amount per unit time of the ultrasonic atomization assembly 20a (sample 4). Table 1 shows corresponding experimental data under the condition that 5ml of aerosol-generating substrate is atomized and the specifications of the atomizer plate 22a/22b and the piezoelectric drive element 21 are the same.

**Table 1 is the experimental comparison data between the ultrasonic atomization assembly of the present disclosure and the existing ultrasonic atomization assembly**

| Serial number | Whether there is a piezoelectric drive element | Whether an atomizer plate has a boss | Whether there is a loading board | Average atomization rate | Average proportion of small-size particles (%) | Noise | Temperature increment (/°C) |
|---|---|---|---|---|---|---|---|
| Sample 1 | Yes | No | No | 3 | 78 | High | 17 |
| Sample 2 | Yes | No | Yes | 7.5 | 82 | Barely audible | 8 |
| Sample 3 | Yes | Yes | No | 4.5 | 63 | High | 17 |
| Sample 4 | Yes | Yes | Yes | 8.1 | 80 | Barely audible | 8 |

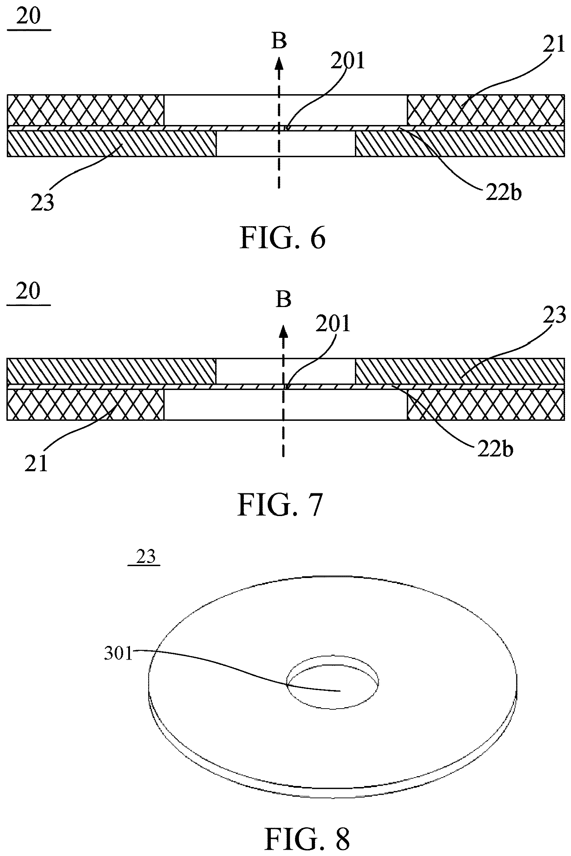

In another alternative embodiment, referring to FIG. 6 or FIG. 7, FIG. 6 is a side sectional view of a first embodiment of an ultrasonic atomization assembly without a boss of the present disclosure; FIG. 7 is a side sectional view of a second embodiment of an ultrasonic atomization assembly without a boss of the present disclosure.

The microporous zone 201 of the atomizer plate 22b is planar. Compared with the ultrasonic atomization assembly 20 (sample 4) corresponding to the boss formed in the microporous zone 201 of the atomizer plate 22b, the ultrasonic atomization assembly 20 (sample 2) corresponding to the alternative embodiment can avoid the problem of an increase in the particle size of the atomized aerosol, the problem is due to an enlarged atomization through hole 201a on the atomizer plate 22b after the boss is stamped, thus the proportion of the small-particle-size aerosol in the atomized aerosol is further improved.

Referring to FIG. 2 and FIG. 8, FIG. 8 is a schematic structural view of a loading board of the present disclosure. The loading board 23 is disposed on a side surface of the atomizer plate 22a/22b which faces away from the piezoelectric drive element 21; the loading board 23 specifically may be closely connected to the atomizer plate 22a/22b by gluing, spot welding, or the like.

Referring to Table 1, the added loading board 23 effectively improves overall structural rigidity of the atomizer plate 22a/22b, changes driving frequency of the atomizer plate 22a/22b, and effectively constrains the vibration mode of the atomizer plate 22a/22b. Besides, the added loading board 23 can constrain the vibration mode of the atomizer plate 22a/22b to a central area of the atomizer plate 22a/22b, thereby not only reducing the invalid vibration of the atomizer plate 22a/22b, but also greatly reducing the noise caused by the invalid vibration of the atomizer plate 22a/22b and the temperature rise of the atomizer plate 22a/22b. Meanwhile, referring to Table 1, the added loading board 23 effectively increases the atomization amount of the aerosol-generating substrate A per unit time, and the proportion of the small-particle-size aerosol in the atomized aerosol. In addition, referring to Table 1, on the basis of adding the loading board 23, the microporous zone 201 of the atomizer plate 22b is made planar, which can further increase the proportion of the small-particle-size aerosol in the atomized aerosol. Furthermore, air bubbles generated during working of the ultrasonic atomization assembly 20 can be reduced.

In the embodiment, the loading board 23 is provided with a second through hole 301, where the second through hole 301 is disposed corresponding to the microporous zone 201 of the atomizer plate 22a/22b, so that the microporous zone 201 of the atomizer plate 22a/22b is at least partially exposed, thereby ensuring that the aerosol-generating substrate A or the atomized aerosol particles can pass through smoothly.

In an alternative embodiment, a ratio of an aperture of the second through hole 301 of the loading board 23 to a diameter of the microporous zone 201 of the atomizer plate 22 is greater than or equal to 0.75 and less than or equal to 1.5; within the ratio range, the vibration mode of the atomizer plate 22 can be effectively constrained, so that the vibration can be concentrated, thereby the invalid vibration and the vibration loss can be reduced, so as to the working noise and the temperature rise of the ultrasonic atomization assembly 20. In an alternative embodiment, the ratio of the aperture of the second through hole 301 to the diameter of the microporous zone 201 is 1. Based on the above solution, the ultrasonic atomization assembly 20 can achieve the best noise reduction effect, and the temperature rise of the ultrasonic atomization 20 is the lowest.

The shape of the loading board 23, the piezoelectric drive element 21 and/or the atomizer plate 22a/22b may be circular; the outer diameter of the loading board 23, the piezoelectric drive element 21 and/or the atomizer plate 22a/22b may also be the same, so as to facilitate subsequent installation and use. In the embodiment, the material of the loading board 23 may be the same as the material of the atomizer plate 22a/22b, and the material may be a rigid metal material such as stainless steel, titanium alloy, palladium alloy and the like, so as to enhance the strength of the ultrasonic atomization assembly 20, and better constrain the vibration mode of the ultrasonic atomization assembly 20.

In an alternative embodiment, the ratio of the outer diameter of the atomizer plate 22a/22b to the thickness of the loading board 23 may be in the range of 23 to 40; in this range, a desired effect of reducing the working noise and temperature rise of the ultrasonic atomization assembly 20 can be achieved, and the vibration loss caused by the invalid vibration can be effectively decreased. In the embodiment, when the outer diameter of the atomizer plate 22 is 10 mm, the thickness of the loading board 23 may range from 0.25 mm to 0.43 mm. In this case 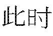 , a better effect of reducing the working noise and temperature rise can be achieved by the ultrasonic atomization assembly 20, and the vibration loss caused by the invalid vibration can be effectively decreased. When the outer diameter of the atomizer plate 22 is 16 mm, the thickness of the loading board 23 may range from 0.4 mm to 0.7 mm. In this case, the ultrasonic atomization assembly 20 can achieve the best effect of reducing the noise and temperature rise.

Referring to FIG. 4, FIG. 4 is a side sectional view of a first embodiment of an ultrasonic atomization assembly with a boss of the present disclosure. In an alternative embodiment, the microporous zone 201 of the atomizer plate 22a raises towards the mist outlet direction B of the ultrasonic atomization assembly 20 to form the boss. The carrier plate 23 is disposed on the liquid inlet surface 221 of the atomizing sheet 22a and is closely combined with the atomizing sheet 22a, and the piezoelectric driving element 21 is disposed on the mist outlet surface 222 of the atomizing sheet 22a and is closely combined with the atomizing sheet 22a.

Referring to FIG. 5, FIG. 5 is a side sectional view of a second embodiment of an ultrasonic atomization assembly with a boss of the present disclosure. In another alternative embodiment, the microporous zone 201 of the atomizer plate 22a raises towards the mist outlet direction B of the ultrasonic atomization assembly 20 to form the boss. The loading board 23 is disposed on the mist outlet surface 222 of the atomizer plate 22a and is closely connected to the atomizer plate 22a, and the piezoelectric drive element 21 is disposed on the liquid inlet surface 221 of the atomizer plate 22a and is closely connected to the atomizer plate 22a.

In the above embodiment, referring to Table 1, the atomizer plate 22a has a boss structure. In the case of the same diameter of the microporous zone 201, the atomizer plate 22a with the boss structure can increase the surface area of the microporous zone 201 to a certain extent, which means the contact area with the aerosol-generating substrate A is increased, thereby the atomization amount per unit time of the ultrasonic atomization assembly 20 can be improved to a certain extent. In the embodiment, the average atomization rate of the atomizer plate 22a with the boss can reach 8.1 mg/s.

Referring to FIG. 6, FIG. 6 is a side sectional view of a first embodiment of an ultrasonic atomization assembly without a boss of the present disclosure. In another embodiment, the microporous zone 201 of the atomizer plate 22b is planar. The loading board 23 is disposed on the side of the liquid inlet surface 221 of the atomizer plate 22b and is closely connected to the atomizer plate 22b, and the piezoelectric drive element 21 is disposed on the side of the mist outlet surface 222 of the atomizer plate 22b and is closely connected to the atomizer plate 22b.

Referring to FIG. 7, different from FIG. 6, the loading board 23 is disposed on the side of the mist outlet surface 222 of the atomizer plate 22a and is closely connected to the atomizer plate 22b, and the piezoelectric drive element 21 is disposed on the side of the liquid inlet surface 221 of the atomizer plate 22b and is closely connected to the atomizer plate 22b. Namely, in FIG. 6 and FIG. 7, the position of the loading board 23 and the position of the piezoelectric drive element 21 are different. In practical disclosure, changing the installation position of the piezoelectric drive element 21 on the atomizer plate 22a/22b can effectively widen the application range of the ultrasonic atomization assembly 20.

In the above alternative embodiment, referring to Table 1, the atomizer plate 22b is the planar atomizer plate without the boss, the structure of the atomizer plate 22b can avoid problem of that the atomization particle size of the atomizer plate increases due to enlarged micro-pores on the atomizer plate after the boss is stamped, and problem of reducing the proportion of small-size particles in the atomized particles. In the above embodiment, the average proportion of small-size particles in the atomized particles of the atomizer plate 22b can reach 82%, which further improves the atomization effect. In the embodiment, in a process of atomizing 5ml of aerosol-generating substrate A, the temperature rise of the ultrasonic atomization assembly 20 with the non-boss atomizer plate 22b is about 8 degrees Celsius, and the working noise is also significantly reduced. During the working process, the average atomization rate of the ultrasonic atomization assembly 20 is 7.5 mg/s, while the average atomization rate of the conventional ultrasonic atomization assembly is 3 mg/s. Therefore, compared with the conventional atomizer plate, the working efficiency of the ultrasonic atomization assembly 20 is also greatly improved.

In the ultrasonic atomization assembly 20 provided by the embodiments of the present disclosure, a loading board 23 is added and placed on a side surface of an atomizer plate 22a/22b away from a piezoelectric drive element 21, which effectively improves overall structural rigidity of the atomizer plate 22a/22b, so that a vibration mode of the atomizer plate 22a/22b is effectively constrained, thus not only decreasing invalid vibration of the atomizer plate 22a/22b, but also greatly reducing noise caused by the invalid vibration of the atomizer plate 22a/22b and a temperature rise of the atomizer plate 22a/22b; meanwhile, the proportion of small-size particles in vaporized particles is effectively increased. Besides, a ratio of an aperture of a second through hole 301 to a diameter of a microporous zone 201 is greater than or equal to 0.75 and less than or equal to 1.5, so that the ultrasonic atomization assembly 20 has a desired noise reduction effect. In addition, the added loading board 23 can reduce a clamping force of a housing 10 and the piezoelectric drive element 21 on the atomizer plate 22a/22b after the installation of the housing 10 and the ultrasonic atomization assembly 20, thus effectively reducing vibration performance attenuation caused by the clamping of the atomizer plate 22a/22b; and sealing reliability of the ultrasonic atomization assembly 20 is increased. Moreover, the second through hole 301 is provided at a position of the loading board 23 corresponding to the microporous zone 201, which can effectively ensure that a liquid inlet surface 221 or a mist outlet surface 222 of the atomizer plate 22a/22b is connected to the external, so as to ensure that an aerosol substrate or atomized aerosol particles can pass through smoothly. Furthermore, the embodiments of the present disclosure add the loading board 23, which can further change the installation position of the piezoelectric drive element 21 on the atomizer plate 22a/22b, thus effectively widening the disclosure range of the ultrasonic atomization assembly 20.

## Claims

1. An ultrasonic atomization assembly (20), comprising:
a piezoelectric drive element (21) provided with a first through hole (211);
an atomizer plate (22a/22b) stacked with the piezoelectric drive element (21), wherein the atomizer plate (22a/22b) is provided with a microporous zone (201) at a position corresponding to the first through hole (211); and
a loading board (23) disposed on the side surface of the atomizer plate (22a/22b) which faces away from the piezoelectric drive element (21), wherein the loading board (23) is provided with a second through hole (301) at a position corresponding to the microporous zone (201);
**characterized in that** the ratio of the aperture of the second through hole (301) to the diameter of the microporous zone (201) is greater than or equal to 0.75 and less than or equal to 1.5, and the loading board (23) is configured to constrain a vibration mode of the atomizer plate (22a/22b) to a central area of the atomizer plate (22a/22b).

2. The ultrasonic atomization assembly of claim 1, wherein the ratio of the diameter of the microporous zone (201) to the aperture of the second through hole (301) is 1.

3. The ultrasonic atomization assembly of claim 1, wherein the microporous zone (201) is planar; or
wherein the microporous zone (201) raises towards the mist outlet direction (B) of the ultrasonic atomization assembly (20) to form a boss.

4. The ultrasonic atomization assembly of claim 1, wherein the microporous zone (201) is located in a central area of the atomizer plate (22a/22b).

5. The ultrasonic atomization assembly of claim 1, wherein a plurality of atomization through holes (201a) is defined in the microporous zone (201), and
wherein an aperture of the atomization through hole (201a) gradually decreases along the mist outlet direction (B) of the ultrasonic atomization assembly (20).

6. The ultrasonic atomization assembly of claim 1, wherein the loading board (23) comprises a metal sheet, and
wherein the thickness of the metal sheet ranges from 0.4 mm to 0.7 mm.

7. The ultrasonic atomization assembly of claim 1, wherein the ratio of the outer diameter of the atomizer plate (22a/22b) to the thickness of the loading board (23) is in the range of 23 to 40.

8. The ultrasonic atomization assembly of claim 1, wherein the atomizer plate (22a/22b) comprises the liquid inlet surface (221) and the mist outlet surface that are disposed opposite to each other, and the loading board (23) is disposed on the liquid inlet surface (221), and the piezoelectric drive element (21) is disposed on the mist outlet surface; or
wherein the atomizer plate (22a/22b) comprises the liquid inlet surface (221) and the mist outlet surface (222) that are disposed opposite to each other, and the loading board (23) is disposed on the mist outlet surface (222), and the piezoelectric drive element (21) is disposed on the liquid inlet surface (221).

9. The ultrasonic atomization assembly of claim 1, wherein the piezoelectric drive element (21), the atomizer plate (22a/22b), and the loading board (23) are all circular and have the same outer diameter;
wherein the atomizer plate (22a/22b) comprises a metallic screen mesh; and
wherein the piezoelectric drive element (21) comprises a piezoelectric ceramic ring.

10. An ultrasonic atomization device, comprising:
a housing (10), comprises a liquid storage cavity (11) and a liquid outlet (12);
an ultrasonic atomization assembly (20) being disposed at the liquid outlet (12), the ultrasonic atomization assembly (20) being configured to atomize an aerosol-generating substrate (A) of the liquid storage cavity (11) when a power is supplied to the ultrasonic atomization assembly (20), wherein the ultrasonic atomization assembly (20) is the ultrasonic atomization assembly (20) as described in any of claims 1 to 9; and
a power supply assembly (30) electrically connected to the ultrasonic atomization assembly (20), the power supply assembly (30) being configured to supply power to the ultrasonic atomization assembly (20).

## Patentansprüche

1. Eine Ultraschallzerstäubungsanordnung (20), umfassend:
ein piezoelektrisches Antriebselement (21), das mit einem ersten Durchgangsloch (211) versehen ist;
eine Zerstäuberplatte (22a/22b), die mit dem piezoelektrischen Antriebselement (21) gestapelt ist, wobei die Zerstäuberplatte (22a/22b) an einer dem ersten Durchgangsloch (211) entsprechenden Position mit einer mikroporösen Zone (201) versehen ist; und
eine Trägerplatte (23), die auf einer dem piezoelektrischen Antriebselement (21) abgewandten Seitenfläche der Zerstäuberplatte (22a/22b) angeordnet ist, wobei die Trägerplatte (23) an einer der mikroporösen Zone (201) entsprechenden Position mit einem zweiten Durchgangsloch (301) versehen ist;
**dadurch gekennzeichnet, dass** das Verhältnis der Öffnungsweite des zweiten Durchgangslochs (301) zum Durchmesser der mikroporösen Zone (201) größer als oder gleich 0,75 und kleiner als oder gleich 1,5 ist und die Trägerplatte (23) dazu ausgebildet ist, einen Schwingungsmodus der Zerstäuberplatte (22a/22b) auf einen zentralen Bereich der Zerstäuberplatte (22a/22b) zu beschränken.

2. Die Ultraschallzerstäubungsanordnung nach Anspruch 1, wobei das Verhältnis des Durchmessers der mikroporösen Zone (201) zur Öffnungsweite des zweiten Durchgangslochs (301) 1 beträgt.

3. Die Ultraschallzerstäubungsanordnung nach Anspruch 1, wobei die mikroporöse Zone (201) eben ist; oder
wobei die mikroporöse Zone (201) in der Nebelaustrittsrichtung (B) der Ultraschallzerstäubungsanordnung (20) hervorsteht, um einen Vorsprung zu bilden.

4. Die Ultraschallzerstäubungsanordnung nach Anspruch 1, wobei die mikroporöse Zone (201) in einem zentralen Bereich der Zerstäuberplatte (22a/22b) angeordnet ist.

5. Die Ultraschallzerstäubungsanordnung nach Anspruch 1, wobei in der mikroporösen Zone (201) eine Vielzahl von Zerstäubungsdurchgangslöchern (201a) ausgebildet ist und
wobei eine Öffnungsweite des Zerstäubungsdurchgangslochs (201a) entlang der Nebelaustrittsrichtung (B) der Ultraschallzerstäubungsanordnung (20) allmählich abnimmt.

6. Die Ultraschallzerstäubungsanordnung nach Anspruch 1, wobei die Trägerplatte (23) ein Metallblech umfasst und
wobei die Dicke des Metallblechs im Bereich von 0,4 mm bis 0,7 mm liegt.

7. Die Ultraschallzerstäubungsanordnung nach Anspruch 1, wobei das Verhältnis des Außendurchmessers der Zerstäuberplatte (22a/22b) zur Dicke der Trägerplatte (23) im Bereich von 23 bis 40 liegt.

8. Die Ultraschallzerstäubungsanordnung nach Anspruch 1, wobei die Zerstäuberplatte (22a/22b) die Flüssigkeitseinlassfläche (221) und die Nebelaustrittsfläche umfasst, die einander gegenüberliegend angeordnet sind, die Trägerplatte (23) auf der Flüssigkeitseinlassfläche (221) angeordnet ist und das piezoelektrische Antriebselement (21) auf der Nebelaustrittsfläche angeordnet ist; oder
wobei die Zerstäuberplatte (22a/22b) die Flüssigkeitseinlassfläche (221) und die Nebelaustrittsfläche (222) umfasst, die einander gegenüberliegend angeordnet sind, die Trägerplatte (23) auf der Nebelaustrittsfläche (222) angeordnet ist und das piezoelektrische Antriebselement (21) auf der Flüssigkeitseinlassfläche (221) angeordnet ist.

9. Die Ultraschallzerstäubungsanordnung nach Anspruch 1, wobei das piezoelektrische Antriebselement (21), die Zerstäuberplatte (22a/22b) und die Trägerplatte (23) alle kreisförmig sind und denselben Außendurchmesser aufweisen;
wobei die Zerstäuberplatte (22a/22b) ein Metallsieb umfasst; und
wobei das piezoelektrische Antriebselement (21) einen piezoelektrischen Keramikring umfasst.

10. Eine Ultraschallzerstäubungsvorrichtung, umfassend:
ein Gehäuse (10), das einen Flüssigkeitsspeicherhohlraum (11) und einen Flüssigkeitsauslass (12) umfasst;
eine Ultraschallzerstäubungsanordnung (20), die an dem Flüssigkeitsauslass (12) angeordnet ist, wobei die Ultraschallzerstäubungsanordnung (20) dazu ausgebildet ist, ein aerosolerzeugendes Substrat (A) des Flüssigkeitsspeicherhohlraums (11) zu zerstäuben, wenn der Ultraschallzerstäubungsanordnung (20) elektrische Energie zugeführt wird, wobei die Ultraschallzerstäubungsanordnung (20) die in einem der Ansprüche 1 bis 9 beschriebene Ultraschallzerstäubungsanordnung (20) ist; und
eine Stromversorgungsanordnung (30), die mit der Ultraschallzerstäubungsanordnung (20) elektrisch verbunden ist, wobei die Stromversorgungsanordnung (30) dazu ausgebildet ist, die Ultraschallzerstäubungsanordnung (20) mit elektrischer Energie zu versorgen.

## Revendications

1. Un ensemble d'atomisation ultrasonique (20), comprenant :
un élément d'entraînement piézoélectrique (21) pourvu d'un premier trou traversant (211) ;
une plaque d'atomisation (22a/22b) empilée avec l'élément d'entraînement piézoélectrique (21), la plaque d'atomisation (22a/22b) étant pourvue d'une zone microporeuse (201) à une position correspondant au premier trou traversant (211) ; et
une plaque de support (23) disposée sur une face de la plaque d'atomisation (22a/22b) tournée à l'opposé de l'élément d'entraînement piézoélectrique (21), la plaque de support (23) étant pourvue d'un deuxième trou traversant (301) à une position correspondant à la zone microporeuse (201) ;
**caractérisé en ce que** le rapport entre le diamètre de l'ouverture du deuxième trou traversant (301) et le diamètre de la zone microporeuse (201) est supérieur ou égal à 0,75 et inférieur ou égal à 1,5, et **en ce que** la plaque de support (23) est configurée pour limiter un mode de vibration de la plaque d'atomisation (22a/22b) à une zone centrale de la plaque d'atomisation (22a/22b).

2. L'ensemble d'atomisation ultrasonique selon la revendication 1, le rapport entre le diamètre de la zone microporeuse (201) et le diamètre de l'ouverture du deuxième trou traversant (301) étant égal à 1.

3. L'ensemble d'atomisation ultrasonique selon la revendication 1, dans lequel la zone microporeuse (201) est plane ; ou
dans lequel la zone microporeuse (201) fait saillie dans la direction de sortie de la brume (B) de l'ensemble d'atomisation ultrasonique (20) pour former un bossage.

4. L'ensemble d'atomisation ultrasonique selon la revendication 1, dans lequel la zone microporeuse (201) est située dans une zone centrale de la plaque d'atomisation (22a/22b).

5. L'ensemble d'atomisation ultrasonique selon la revendication 1, dans lequel une pluralité de trous traversants d'atomisation (201a) est ménagée dans la zone microporeuse (201), et
dans lequel la dimension de l'ouverture du trou traversant d'atomisation (201a) diminue progressivement suivant la direction de sortie de la brume (B) de l'ensemble d'atomisation ultrasonique (20).

6. L'ensemble d'atomisation ultrasonique selon la revendication 1, dans lequel la plaque de support (23) comprend une tôle métallique, et
dans lequel l'épaisseur de la tôle métallique est comprise entre 0,4 mm et 0,7 mm.

7. L'ensemble d'atomisation ultrasonique selon la revendication 1, dans lequel le rapport entre le diamètre extérieur de la plaque d'atomisation (22a/22b) et l'épaisseur de la plaque de support (23) est compris entre 23 et 40.

8. L'ensemble d'atomisation ultrasonique selon la revendication 1, dans lequel la plaque d'atomisation (22a/22b) comprend la surface d'entrée de liquide (221) et la surface de sortie de la brume qui sont disposées à l'opposé l'une de l'autre, la plaque de support (23) est disposée sur la surface d'entrée de liquide (221) et l'élément d'entraînement piézoélectrique (21) est disposé sur la surface de sortie de la brume ; ou
dans lequel la plaque d'atomisation (22a/22b) comprend la surface d'entrée de liquide (221) et la surface de sortie de la brume (222) qui sont disposées à l'opposé l'une de l'autre, la plaque de support (23) est disposée sur la surface de sortie de la brume (222) et l'élément d'entraînement piézoélectrique (21) est disposé sur la surface d'entrée de liquide (221).

9. L'ensemble d'atomisation ultrasonique selon la revendication 1, dans lequel l'élément d'entraînement piézoélectrique (21), la plaque d'atomisation (22a/22b) et la plaque de support (23) sont tous de forme circulaire et présentent le même diamètre extérieur ;
dans lequel la plaque d'atomisation (22a/22b) comprend un tamis métallique ; et
dans lequel l'élément d'entraînement piézoélectrique (21) comprend un anneau en céramique piézoélectrique.

10. Un dispositif d'atomisation ultrasonique, comprenant :
un boîtier (10) comprenant une cavité de stockage de liquide (11) et une sortie de liquide (12) ;
un ensemble d'atomisation ultrasonique (20) disposé au niveau de la sortie de liquide (12), l'ensemble d'atomisation ultrasonique (20) étant configuré pour atomiser un substrat générateur d'aérosol (A) de la cavité de stockage de liquide (11) lorsque de l'énergie électrique est fournie à l'ensemble d'atomisation ultrasonique (20), dans lequel l'ensemble d'atomisation ultrasonique (20) est l'ensemble d'atomisation ultrasonique (20) tel que décrit dans l'une quelconque des revendications 1 à 9 ; et
un ensemble d'alimentation électrique (30) connecté électriquement à l'ensemble d'atomisation ultrasonique (20), l'ensemble d'alimentation électrique (30) étant configuré pour fournir de l'énergie électrique à l'ensemble d'atomisation ultrasonique (20).
